# EUROPEAN PATENT SPECIFICATION

(11) **EP 0 775 216 B1**
(45) Date of publication and mention of the grant of the patent: **03.04.2002**
(21) Application number: 95927015.8
(22) Date of filing: 26.07.1995
(51) Int. Cl.: C12Q 1/68, C12Q 1/70

(54) **OLIGONUCLEOTIDES SPECIFIC FOR HCV AND THEIR USE**
FÜR HCV SPEZIFISCHE OLIGONUKLEOTIDE UND DEREN VERWENDUNG
OLIGONUCLEOTIDES SPECIFIQUES DU VHC ET LEUR UTILISATION

(30) Priority: 27.07.1994 GB 9415129
(43) Date of publication of application: 28.05.1997
(73) Proprietor: CAMBRIDGE UNIVERSITY TECHNICAL SERVICES LIMITED, Cambridge CB2 1TS (GB)
(72) Inventor: PETRIK, Juraj, Cambridge CB2 5QL (GB); ALLAIN, Jean-Pierre, Herst SG8 7TG (GB)
(74) Representative: Perry, Robert Edward
(86) International application number: GB9501768
(87) International publication number: WO9603528

(56) References cited:
- EP-A- 0 331 939
- EP-A- 0 374 665
- EP-A- 0 435 150
- WO-A-88/01302
- WO-A-89/08146
- WO-A-91/18117
- WO-A-92/03127
- WO-A-93/04199
- WO-A-93/13225
- WO-A-93/25563
- WO-A-94/08032
- WO-A-94/12657
- WO-A-94/16108
- JOURNAL MEDICAL VIROLOGY, vol. 42, 1994 pages 22-28, VAN DOORN ET AL cited in the application

## Description

### Field of the Invention

This invention relates to oligonucleotides and their use, especially in extraction and assay procedures, and also to apparatus and methods associated therewith.

### Background of the Invention

Hepatitis C virus (HCV) was cloned in 1989. The development of first tests based on recombinant antigens to detect anti-HCV antibodies confirmed what has been long suspected: that the vast majority of post-transfusion and sporadic non-A, non-B hepatitis is caused by HCV and frequently develops into chronic hepatitis, cirrhosis and hepatocellular carcinoma.

Mandatory anti-HCV testing of blood donations started in the UK in 1991. Common practice in antiviral testing includes a cheap, less specific but sensitive test as a screening method, and a more sophisticated specific method for confirmation. Wherever possible, these tests should be complementary in the sense that one of them detects viral antigen(s) and the other detects antibodies against the virus. In some cases, however, antigen detection is very difficult or below the sensitivity of current methods. This is the situation with HCV; its concentration in serum is much lower than of other blood-borne viruses. In this case, the polymerase chain reaction (PCR) may be used to amplify a genomic fragment many million times in hours.

PCR amplifies a fragment of DNA flanked by the primers for the amplification. When the template for amplification is RNA, it needs to be first transcribed into a DNA copy using the enzyme reverse transcriptase (RT). Because RNA molecules are more fragile and sensitive to degradation than DNA, and the efficiency of RT is rather low, this is the most vulnerable step of the whole procedure of RT-PCR.

RNA extraction has been much improved by the introduction of strong denaturation agents such as guanidine-isothiocyanate in the extraction protocol. Combined with single acid-phenol/chloroform extraction, this is the most commonly used RNA extraction procedure. A disadvantage is that it uses centrifugation, pipetting the supernatant into a new tube, precipitation, centrifugation and pellet washing, which are all labour-intensive procedures.

Recent methods include the utilisation of the strong reaction between streptavidin and biotin in combination with paramagnetic particle technology. Briefly, a specific probe for desired RNA (usually oligo dT 12-18, which is complementary to poly(A) tail described for most messenger RNA molecules) is biotinylated, and paramagnetic particles (PMP) are coated with streptavidin. All molecules captured through complementary annealing to the biotinylated probe are then bound to PMP through irreversible biotin-streptavidin binding, and can be washed and eluted in a form ready for subsequent reactions. Van Doorn *et al*, J. Med. Virol. 38:298-304 (1992) and J. Med. Virol. 42:22-28 (1994) adapted this method for HCV RNA extraction, using biotinylated capture oligonucleotides from various coding regions of HCV genome. However, the variability of HCV is high (at least 6 types described on the bases of nucleotide sequences of which type 1, 2 and 3 are common in UK), and using sequence specific oligonucleotides would mean either type-specific extraction (too expensive) or the danger of missing some variants.

The HIV and HTLV genome consists of a single-stranded RNA molecule (two identical in one virus particle) of plus polarity. The length of the RNAs is between 9 and 10 kb and they contain a poly(A) stretch at their 3' end. As this is a common feature of a vast majority of eukaryotic messenger RNA (mRNA) molecules, it does not provide a high degree of selection; specific oligonucleotides suitable for the capture of genomic RNAs come from different part of the genome.

HIV and HTLV are retroviruses. Thus, the first step in the genome's replication is the synthesis of minus strand DNA by the virus-coded enzyme reverse transcriptase. As for most other polymerases, this enzyme needs a primer to start the synthesis. In the case of retroviruses, the primer is provided by the 3'-end of particular transfer RNA (tRNA) molecules. The retroviral genomic RNA contains a sequence of 18 nucleotides complementary to the last 18 nucleotides of corresponding tRNA, called the primer binding site (PBS). The mutation rate of retroviruses is very high, as a consequence of a low fidelity of most reverse transcriptases. However, PBS has to be preserved in any replication-competent virus and is therefore an ideal target sequence for designing the capture oligonucleotide. Various retroviruses differ in the use of tRNA primer molecules and several retroviruses can have the same primer tRNA (for example, all avian retroviruses use tryptophan tRNA, all murine except MMTV use proline tRNA etc.). Currently, only 5 human retroviruses are recognised, i.e. HIV 1 and 2, both primed with lysine3 tRNA; HTLV 1 and 2, both primed with proline tRNA; and human spumaretrovirus (HSRV), primed with lysine 1,2 tRNA.

There are several ways of detecting PCR products. For example, the product is run on agarose gel and the band visualised by ethidium-bromide staining. To increase the low sensitivity, and include specificity, the gel can be used for Southern blotting, and nucleic acid transferred to a membrane detected by hybridisation. This is sensitive and specific, but time-consuming and inconvenient for routine work. These disadvantages can be overcome to some extent by various ELISA-based protocols using antibodies against labels used to label PCR primers and/or probes. As they do not use gel electrophoresis, at least some of the steps can be automated. These protocols are usually sufficiently sensitive and specific, but they still include multistep post-PCR processing.

Many protocols avoiding gel electrophoresis use the biotin-streptavidin system for the capture and washing of PCR product from non-specificities. Annealing or labelled probe to washed PCR product should provide a sensitive and specific method to detect and to quantitate the PCR product. In fact, there is one more obstacle to remove: the PCR product (with the exception of asymmetric PCR which is not widely used) is double-stranded, and the strands need to be separated before annealing to the probe either by heat or by alkali denaturation. This not only poses technical problems but, as a result of competition between the probe and the second strand of the PCR product, the numerical expression of results obtained by ELISA-based methods or other methods involving liquid hybridisation do not correspond to amounts of product seen on ethidium-bromide stained gel, or indeed, after hybridisation to Southern blot.

### Summary of the Invention

According to a first aspect of the invention, one novel oligonucleotide probe is polydA, comprising at least 20 deoxyadenosine residues.

Such a probe, conjugated to a capture moiety such as biotin, can be used for HCV RNA capture. It may also be used for capturing RNA of any similar type, i.e. having a poly(U) sequence, of 10 or more U residues, at the 3' end or anywhere in the genomic DNA. The method is type-independent and specific.

### Description of the Drawings

The accompanying drawings are for the purpose of illustration only. In the drawings:
Fig. 1 is a diagrammatic representation of two complementary nucleotide sequences; and
Fig. 2 is a sectional side view of an embodiment of apparatus of the invention.

### Description of the Invention

Evidence has been obtained that a stretch of U and not A residues is present on the 3'-end of HCV RNA, and that oligonucleotide dA40 can be safely used for the extraction of HCV, regardless of type variations. Apparently, only HCV RNA, and RNA of a few related viruses (e.g. GBV A, B, C), contains a 3'-polyU stretch.

Further, the novel probe can be used in a new method of full-length HCV RNA extraction, simultaneously from many, e.g. 60, samples. The basis for a fast and efficient RT PCR procedure, in the detection of serum HCV RNA, comprises the following steps:
1. RNA extraction.
2. Combined cDNA/1st PCR.
3. 2nd, nested PCR.
4. Detection of the PCR product.

To make RT-PCR suitable for diagnostics, the RNA extraction method must be safe and simple but at the same time able to process many samples. For this purpose, a diagnostic plate of the type normally used for antibody detection, but modified in accordance with the invention, was used. A magnetic platform accommodates the diagnostic plate and, to make the extraction safer, a protection device, of a transparent plastics material is provided, to expose only one row of wells at a time, thus reducing or preventing aerosol contamination.

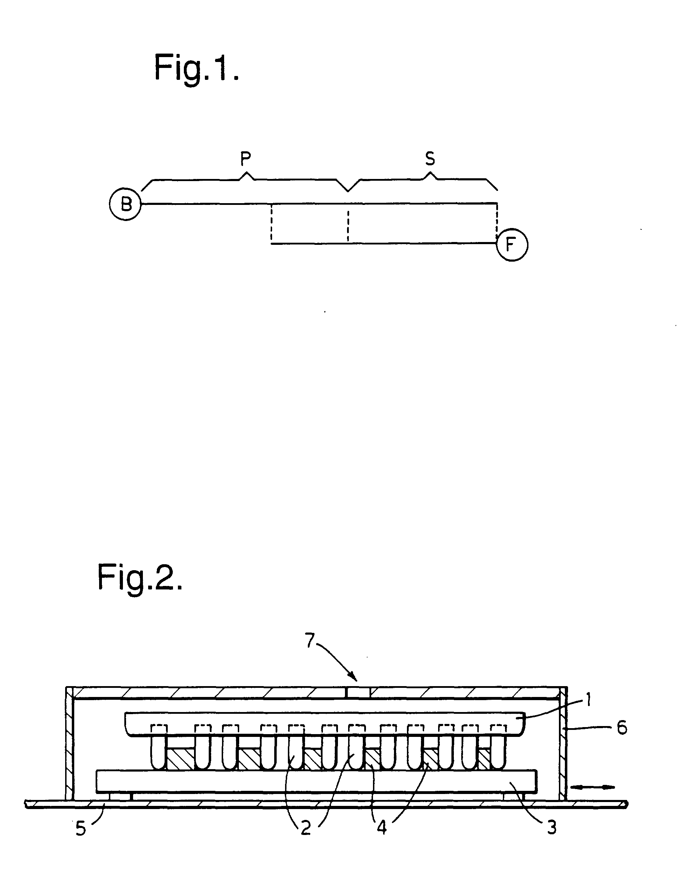

Such a modified diagnostic device is shown in Fig. 2. It comprises an extraction plate 1 having an array of 12 x 5 wells 2. The plate and wells are mounted on a stand 3, e.g. of a plastics material, with fixed magnetic rods 4. The stand 3 is on a base 5. A shield 6, e.g. of a plastics material, is moveable, as shown by the arrows, with respect to the remainder of the device. It includes an opening at 7, through which one row of 5 wells 2 is visible.

Similar protection devices have been designed, e.g. of transparent plastics material, for the handling of tubes for the 1st and 2nd PCR. The method has been validated using coded panel of samples from the second Eurohep proficiency study. The sensitivity has been shown to be the same as for a commercially-available assay (Amplicor, Roche) for the dilution panel of type 1 sequence, but the novel method was superior to others in detection of dilution panel of type 3 sequence.

The protocol in its present form (for 60 samples) can be completed within an 8-hour working day by a single trained person. Alternatively, the RNA extraction can be separated from the amplification and detection, making the process even more efficient. The procedure is suitable for routine RT-PCR diagnostics of HCV RNA. It has been designed in a way that it can be easily automated, increasing the effectivity of the method.

The advantages of new RNA extraction protocol are that it is selective for HCV RNA, fast (at least 4-5 times faster than any other HCV extraction protocol), and simple (it avoids centrifugations, organic solvent extractions, transfers between tubes or wells, precipitations, or use of toxic chemicals such as guanidine-isothiocyanate). Further, it provides RNA ready for cDNA synthesis. RNA is eluted in small volumes of the mixture of water/DMSO, so that even initial denaturation before cDNA synthesis is unnecessary.

Full-length RNA is extracted. This is shown by consistent amplification of non-coding region (NCR) sequences located at the 5' end, although the extraction method is 3' end-specific, for the poly(U) stretch. This provides an opportunity to amplify any part of the HCV genome.

Fluorescent detection of the PCR product is preferable to gel electrophoresis for several reasons. In particular, it is faster and simpler, and requires much less manipulation. It provides an additional check for specificity of amplification, as it includes annealing to an internal probe. It also provides an opportunity for automation. Further, the inclusion of a set of the dilutions of an *in vitro*-transcribed RNA, as a control, added to uninfected serum, allows the quantitation of PCR products.

In one aspect of this invention, the known competition between the probe and the second strand of the double-stranded PCR product is reduced or avoided by digesting all parts of the PCR products except for that part relevant to the assay. The necessary treatment includes the use of the enzyme uracyl-N-glycosidase (UNGase). This enzyme is normally used for carry-over prevention when dUTP instead of dTTP is used in PCR reactions. Contaminating DNA from previous amplifications can be removed from newly assembled reactions by UNGase treatment. The enzyme is then heat-denatured, template is added and PCR performed. In this invention, there is a second treatment with UNGase, after the PCR has finished. As UNGase cuts DNA at each U-residue this approach is viable only when using a pair of primer probe sequences with probe sequence complementary to (or partially overlapping) the sequence immediately following the primer sequence. This sequence must be free of U-residues (minimum of 10-nucleotide U-free sequence).

Fig. 1 shows such a pair of primers. There is a deoxynucleotide primer (P) having a biotin (B) capture moiety at its 5' end and, at its 3' end, a sequence (S) not containing any T residues. There is also a complementary probe having fluorescein or another fluorescent label (F) at its 5' end.

Primer together with immediately following sequence will be left intact after post-PCR UNGase treatment, while most of PCR product will be fragmented. Only mild heat treatment is necessary to dissociate small fragments left after UNGase treatment. Fluorescein-labelled probe is annealed to this sequence at a suitable temperature; because primer is biotinylated at its 5'-end, this complex can be captured on streptavidin-coated paramagnetic particles, washed to remove non-specific binding and measured using flow cytometer or fluorometer. An additional advantage of this protocol is that the possibility of carry-over is further limited.

A linear relation is obtained, between fluorescence measured and the amount of PCR product (as determined by Southern blot hybridisation). This assay can thus be used for quantitation. A part of the HCV genome stretching from 5'-NCR to first third of El gene (about 1 kb) has been amplified, the fragment cloned in TA vector, the sense strand tailed with homopolymeric T-tail, and subcloned in a vector allowing *in vitro* transcription. RNA of defined size is produced which carries U-tail on its 3'-end mimicking the genomic RNA. This control RNA is measured and dilutions containing known numbers of RNA molecules are added to negative serum and processed in parallel to other samples.

Except for minor differences in methodology that will readily be within the practice of the skilled man, the other probes defined herein can be used in similar fashion to the illustrative HCV probe. In general, the following steps are used for the extraction of HCV, and optionally HIV and HTLV:
I. Preparation of streptavidin (or equivalent) coated paramagnetic particles (SPMP) with attached capture oligonucleotides (sequences given above; see below for example)
II. Extraction of HCV, HIV, HTLV RNA separately or simultaneously (see protocols below).

The following specific description illustrates the various aspects of the invention.

Mixes are prepared in a separate room. RNA extraction and set-up of the combined cDNA/RT PCR is done on hood 1. Transfer of 1 µl of the 1st PCR to the tubes for 2nd PCR and aliquoting 2nd PCR mix are done in hood 2.

The PCR products are never handled in the same containment used for RNA extraction or 1st or 2nd PCR set-up. Only plugged tips are used throughout the procedure. In addition, the illustrated, simple plexiglass shielding device is used for simultaneous RNA extraction from 60 samples, and handling the 1st and 2nd PCR tubes. Only one row of 5 tubes is exposed at a time, minimising the possibility of aerosol contamination and at the same time helping to localise samples to be processed.

The following abbreviations are used:
- AP 60: 60-well diagnostic Abbott plate used for RNA extraction
- EXP 5: Electrapette Exp, 5 channel, 2-125 µl for use with Abbott plate and 96-well plate (NBL)
- PRO 8C: Proline 8-channel manual pipette, 5-50 µl
- DINC: Dynamic Incubator Commander (Abbott)
- ANA 8C/10: Anachem manual multichannel pipette, 1-10 µl
- OS: Orbital shaker
- MAG 60: Novel magnetic stand similar to AP 60
- PMP/S: Paramagnetic particles coated with streptavidin (Promega)
- BIO: Biotin
- FLU: Fluorescein
- RES: Reservoir: sterile tubes/Aliquoting plates
- dA40: The following sequence:

AAAAAAAAAA AAAAAAAAAA AAAAAAAAAA AAAAAAAAAA

The following buffers are used for RNA extraction and for fluorescence detection and quantitation:
1. 10 x lysis buffer:
   - 0.1: M Tris-Cl, pH 7.4
   - 1.4: M NaCl
   - 0.05: M KCl
   - 10%: Triton X-100
   Add dithiotretol (DTT) to 50 mM and RNAsin to 20 U per sample just before use.
2. 5 x binding buffer:
   - 0.05: M Tris-Cl, pH 7.4
   - 2.5: M LiCl
   - 0.01: M EDTA
3. 0.5 x SSC:
   - 0.075: M NaCl
   - 0.0075: M sodium citrate, pH 7.0
4. 1 x PCR buffer: Perkin-Elmer

### I. Preparation of SPMP with attached capture oligonucleotides

This procedure is for Promega SPMP (referred to below as "particles"). Dynal® particles can also be used.

Particles are prepared in bulk. They can be prepared in advance and stored in a refrigerator for several days.

In case a mix of particles with different attached capture oligonucleotides is used, they can be prepared separately or together. In any case, however, they should be resuspended in 30 µl of 5 x binding buffer per sample for protocols II and VI below; they should be resuspended in 150 µl of 2 x binding buffer for protocol VII.
a) Concentrate appropriate amount of particles (100 µl per capture oligonucleotide per sample) using magnetic concentrator (MC).
b) Discard supernatant and resuspend particles in 0.5 x SSC. Concentrate. Repeat once more.
c) Add 50 pMoles of capture oligonucleotide per sample (in bulk, diluted in 0.5 x SSC).
d) Incubate 3 minutes at room temperature with shaking.
e) Wash 1 x with 0.5 x SSC using MC.
f) Wash 1 x with 5 x binding buffer using MC.
g) Resuspend in appropriate volume of 5 x binding buffer (30 µl per sample, procedures VI and VII) or 2 x binding buffer (150 µl of 2 x binding buffer, procedure VIII).

### II. Extraction of HCV RNA

1. Mix appropriate amount of particles with capture oligo dA40 prepared as described in V (30 µl per sample) with corresponding amount of 10 x lysis buffer (15 µl per sample).
2. Add 45 µl of this mixture to each plasma/serum sample (105 µl) which were pipetted beforehand into 60-well Abbott diagnostic plate or equivalent.
3. Incubate 10 minutes at 37°C with shaking.
4. Concentrate using MAG 60. Discard supernatant.
5. Resuspend particles in 0.5 x SSC by pipetting up and down. Shake at room temperature. Concentrate on MAG 60. Discard supernatant.
6. Repeat 5.
7. Resuspend each sample in 13 µl of DMSO/DEPC-treated water.
8. Elute annealed RNA. Incubate 5 minutes at 45°C with shaking.
9. Concentrate on MAG 60. Transfer 12 µl of eluate to System 9600 MicroAmp tubes (Applied Biosystems). At this stage RNA can be used directly in a combined cDNA/PCR reaction (by simply adding 8 µl of reaction mix) or can be frozen.

### III. cDNA/1st PCR

1. Pipette in reaction mixture (8 µl) from aliquoting plate to 9600 tubes over 5' at rt using PRO 8C.
2. Transfer reaction tubes in the 9600 system (for cDNA synthesis and 1st PCR).
3. Incubate for 30' at 37°C (cDNA synthesis).
4. Incubate
   - 3': 95°C
   - 25': 48°C.
5. Incubate
   - 30": 72°C
   - 20": 95°C
   - 25": 48°C (30 cycles, 2 h 15').
6. Incubate
   - 7': 72°C (final extension).
   - hold: 4°C for 8 h.
7. Transfer aliquots of 1st PCR reaction (1 µl) from 9600 tubes 1st set to 9600 tubes 2nd set over 10" at rt using ANA 8C/10.

### IV. 2nd (nested) PCR

1. Pipette in 2nd PCR mix (19 µl) from aliquoting plate to 2nd set of 9600 tubes containing µl of 1st PCR over 5' at rt using PRO 8C.
2. Incubate
   - 10': RT
   - 10': 98°C.
3. Incubate
   - 25": 48°C.
4. Incubate
   - 30": 72°C
   - 20": 95°C
   - 25": 48°C (30 cycles, 2 h 15').
5. Incubate
   - 7': 72°C (Final extension).

These protocols II, III and IV are suitable for automation, e.g. using automatic dispensers where appropriate. After step 1 of protocol IV, aliquoting, UV treatment, freezing and storing may be carried out.

### V. Detection of Product

1. Pipette in 2nd PCR (A-Z) from 9600 2nd set to 96 well plate 5' at rt using PRO 8C.
2. Pipette in UNGase = 10 µl of Dynal® PMP in 1 x binding buffer containing F5040 (FLU) (18-19 µl) from aliquot plate to 96 well plate over 5' at rt using PRO 8C.
3. Incubate 96 well plate (20 µl) for 1-2 hrs at 34°C using 9600 system.
4. Incubate 96 well plate (20 µl) for 2' at 75°C using 9600 system.
5. Incubate 96 well plate (20 µl) for 5' at 55°C using 9600 system.
6. Concentrate for 1' at 55°C using MAG 96 well plate PCR block.
7. Pipette out 20 µl from 96 well plate to waste over 3' at 55°C.
8. Pipette in 0.5 x SSC (50 µl) from aliquot plate to 96 well plate over 3' at rt using PRO 8C.
9. Wash 96 well plate for 3' at rt using VAR I.
10. Concentrate for 1' at rt using MAG 96.
11. Pipette out 50 µl from 96 well plate to waste 3' at rt using PRO 8C.
12-15 Repeat steps 8-11.
16. Resuspend in 100-200 ml 0.5 x SSC; transfer to plastic tubes for flow cytometer; assay using flow cytometer.

## Claims

1. An oligonucleotide probe that is or comprises polydA of at least 20 deoxyadenosine residues.

2. A probe according to claim 1, conjugated to a capture moiety.

3. A probe according to claim 2, wherein the capture moiety is biotin.

4. Use of a probe according to claim 2 or claim 3, in a serum sample, for the capture of RNA of HCV, HTLV or other viral RNA including a sequence having sufficient complementarity to the probe.

## Patentansprüche

1. Testsubstanz aus einem Oligonucleotid, das polydA von mindestens 20 Desoxydenosinresten ist oder umfaßt.

2. Testsubstanz gemäß Anspruch 1, konjugiert zu einer Einfangkomponente.

3. Testsubstanz gemäß Anspruch 2, wobei die Einfangkomponente Biotin ist.

4. Verwendung einer Testsubstanz gemäß Anspruch 2 oder Anspruch 3 in einer Serumprobe zum Einfangen der RNS von Hepatitis-C-Viren, HTLV-Viren, oder einer anderen viralen RNS mit einer Abfolge mit ausreichender Komplementarität zu der Testsubstanz.

## Revendications

1. Sonde oligonucléotidique qui est ou qui comprend une polydA d'au moins 20 résidus désoxyadénosine.

2. Sonde selon la revendication 1 conjuguée à une entité de capture.

3. Sonde selon la revendication 2 où l'entité de capture est la biotine.

4. Utilisation d'une sonde selon la revendication 2 ou la revendication 3 dans un échantillon de sérum pour la capture d'ARN de HCV, HTLV ou d'un autre ARN viral incluant une séquence ayant une complémentarité suffisante à l'égard de la sonde.
